# EUROPEAN PATENT APPLICATION

(11) **EP 4 026 582 A1**
(43) Date of publication of application: **13.07.2022**
(21) Application number: 20872735.4
(22) Date of filing: 07.09.2020
(51) Int. Cl.: A61M 25/00

(54) **CATHETER**

(30) Priority: 30.09.2019 JP 2019179471
(71) Applicant: TERUMO Kabushiki Kaisha, Tokyo 151-0072 (JP)
(72) Inventor: OKAMURA, Ryo, Ashigarakami-gun, Kanagawa 259-0151 (JP); YASUNAGA, Mitsuteru, Fujinomiya-shi, Shizuoka 418-0015 (JP)
(74) Representative: Casalonga
(86) International application number: PCT/JP2020/033839
(87) International publication number: WO 2021/065364

(57) **Abstract**

Provided is a catheter (1) having a tubular body (2), wherein: the tubular body (2) comprises a base section (23) and a shaping section (24); the shaping section (24) comprises a first bending section (25), a first middle section (26) that is further to the tip-end side than the first bending section (25), a second bending section (27) that is further to the tip-end side than the first middle section (26), a second middle section (28) that is further to the tip-end side than the second bending section (27), a third bending section (29) that is further to the tip-end side than the second middle section (28), and a distal-end section (30) that is further to the tip-end side than the third bending section (29); the first bending section (25) and the first middle section (26) are located on a first plane (P1); the second bending section (27) and the second middle section (28) are located on a second plane (P2) different from the first plane (P1); the angle (αd) formed by the first middle section (26) with respect to an extension line extending in the tip-end direction from a site on the tip-end side of the base section (23) is more than 0° and less than 90°; and the axial center of the distal-end section (30) is separated from the extension of the line of the axial center of the second middle section (28) .

## Description

### Technical Field

The present invention relates to a catheter to be inserted into a body lumen.

### Background Art

Currently, intervention is performed to treat a lesion area such as a heart, blood vessels, a liver, a brain, digestive organs, and urinary organs with a long catheter inserted into a blood vessel through a hole in skin.

For example, when a lesion area is present in the liver, a therapeutic catheter inserted up to the vicinity of a hepatic artery may be used to supply an embolic agent or a drug to the lesion area. In such a treatment, it is desirable to use a guiding catheter such that the therapeutic catheter is capable of fully exhibiting a function at a target position. The guiding catheter is capable of alleviating a reaction caused by the insertion of the therapeutic catheter and applying a backup force to the therapeutic catheter so as to hold the therapeutic catheter at a desired position.

For example, PTL 1 describes a catheter having a distal end portion wound on a surface perpendicular to a substantially linear base portion on a distal side of the base portion.

### Citation List

### Patent Literature

PTL 1: US Patent No. 4,169,464 specification

### Summary of Invention

### Technical Problem

The catheter may pass through, before reaching a target site, bent portions such as a merging portion or a branching portion of a blood vessel, or a sharply bent blood vessel. The catheter needs to pass such a bent portion and then be oriented to the blood vessel ahead of the bent portion.

The present invention is made to solve above problems, and an object thereof is to provide a catheter that has high transmissibility of a pushing force, and is capable of being oriented in a desirable direction in a blood vessel ahead of a bent blood vessel.

### Solution to Problem

To achieve the above object, there is provided a catheter including a tubular body that communicates from a proximal end to a distal end, in which the tubular body includes a substantially linear base portion and a shaped portion connected to a distal side of the base portion, and the shaped portion includes a first bending portion that is bent at a distal side relative to the base portion, a first intermediate portion that is located on a distal side relative to the first bending portion, a second bending portion that is bent at a distal side relative to the first intermediate portion, a second intermediate portion that is located on a distal side relative to the second bending portion, a third bending portion that is bent at a distal side relative to the second intermediate portion, and an extreme distal end portion that is located on a distal side relative to the third bending portion and has a distal end opening formed therein, the first bending portion and the first intermediate portion are located on a first plane, the second bending portion and the second intermediate portion are located on a second plane different from the first plane, an angle formed by the first intermediate portion with respect to an extension line extending from a portion of the base portion on the distal side to a distal direction is more than 0° and less than 90°, and an axis of the extreme distal end portion is separated from an extension line of an axis of the second intermediate portion.

### Advantageous Effect of Invention

In the catheter configured as described above, since the angle formed by the first intermediate portion with respect to the extension line extending from the portion of the base portion on the distal side to the distal direction is more than 0° and less than 90°, it is easy to transmit a pushing force to the distal side. Since the axis of the extreme distal end portion is separated from the extension line of the axis of the second intermediate portion, the extreme distal end portion may be oriented, in a blood vessel ahead of a bent portion of the blood vessel, in a desirable direction different from a direction in which the second intermediate portion is oriented.

The third bending portion may be bent to a side away from the first plane from a proximal side toward the distal side. Accordingly, the catheter can be oriented in a desirable direction with respect to a blood vessel oriented in a predetermined direction in a blood vessel ahead of the bent portion of the blood vessel. Since the third bending portion is bent to the side away from the first plane, the catheter is likely to transmit the pushing force from the proximal side to the distal end of the catheter, as compared with a case where the catheter is bent to a side closer to the first plane. When the third bending portion is bent to the side away from the first plane, the third bending portion is likely to have a structure of being bent to a side substantially opposite to the second bending portion. Therefore, when the second bending portion passes the bent portion of the blood vessel, the extreme distal end portion can be oriented to a side substantially opposite to a bending direction of the bent portion of the blood vessel. Therefore, for example, when the blood vessel ahead of the bent portion of the blood vessel is bent in a direction different from the bending direction of the bent portion, the extreme distal end portion can be oriented in a desirable direction with respect to the blood vessel ahead of the bent portion.

The third bending portion may be bent to a side where the base portion is located with respect to the second plane, from the proximal side toward the distal side. Accordingly, when the first bending portion, the first intermediate portion, and the second bending portion are deformed so as to draw a three-dimensional spiral in the blood vessel, the extreme distal end portion oriented by the third bending portion can be oriented in a desirable direction with respect to the blood vessel oriented in a predetermined direction.

The third bending portion may be bent to a side opposite to a side where the base portion is located with respect toward the second plane, from the proximal side toward the distal side. Accordingly, when the first bending portion, the first intermediate portion, and the second bending portion are deformed so as to draw a three-dimensional spiral in the blood vessel, the extreme distal end portion oriented by the third bending portion can be oriented in a desirable direction with respect to the blood vessel oriented in a predetermined direction ahead of the bent portion of the blood vessel.

A direction in which the second bending portion is bent is a counterclockwise direction from the proximal end to the distal end when the catheter is viewed from the distal side in parallel to an extending direction of the base portion. Accordingly, the catheter inserted from an artery of an arm may pass the bent portion of the blood vessel by utilizing counterclockwise bending of the second bending portion. Further, an operator can effectively transmit a rotational force to the shaped portion of the catheter by applying the rotational force to the proximal end portion of the catheter in a direction (a counterclockwise direction) in which the second bending portion is bent to the distal side. When a three-dimensional shaped portion of the catheter rotates, the catheter can easily pass the bent portion of the blood vessel.

A direction in which the second bending portion is bent is a clockwise direction from the proximal end to the distal end when the catheter is viewed from the distal side in parallel to an extending direction of the base portion. Accordingly, the catheter inserted from an artery of a foot can pass the bent portion of the blood vessel by utilizing clockwise bending of the second bending portion. Further, the operator can effectively transmit a rotational force to the shaped portion of the catheter by applying the rotational force to the proximal end portion of the catheter in a direction (a clockwise direction) in which the second bending portion is bent to the distal side. When the three-dimensional shaped portion of the catheter rotates, the catheter can easily pass the bent portion of the blood vessel.

A minimum radius of curvature of the second bending portion may be smaller than a minimum radius of curvature of the first bending portion, and a minimum radius of curvature of the third bending portion may be smaller than the minimum radius of curvature of the second bending portion. Therefore, when the catheter is proceeded from a left subclavian artery to a descending aorta, the first bending portion is in contact with an upper side of the left subclavian artery, and the second bending portion is in contact with a blood vessel wall of an aortic arch on an ascending aorta side and a chest side. Since the entire shaped portion is a stable state and the third bending portion is oriented to a dorsal side of the descending aorta in a state of being floated in an aorta, the catheter can be easily proceeded to the descending aorta without a guide wire.

### Brief Description of Drawings

[Fig. 1] Fig. 1 is a plan view illustrating a catheter according to a first embodiment.
[Fig. 2] Fig. 2 is a diagram illustrating a distal end portion of the catheter according to the first embodiment, in which (A) is a plan view, (B) is a view as seen from an arrow B in (A), and (C) is a view as seen from an arrow C in (A).
[Fig. 3] Fig. 3 is a schematic diagram illustrating an arrangement of the catheter approached from a femoral artery in a blood vessel.
[Fig. 4] Fig. 4 is a schematic diagram illustrating a state where the catheter approached from the femoral artery is inserted into a celiac artery, in which (A) is a side view, and (B) is a cross-sectional view along a D-D line in (A).
[Fig. 5] Fig. 5 is a plan view illustrating a catheter according to a second embodiment.
[Fig. 6] Fig. 6 is a diagram illustrating a distal end portion of the catheter according to the second embodiment, in which (A) is a plan view, (B) is a view as seen from an arrow B in (A), and (C) is a view as seen from an arrow C in (A).
[Fig. 7] Fig. 7 is a schematic diagram illustrating an arrangement of the catheter approached from an artery of a left arm in a blood vessel.
[Fig. 8] Fig. 8 is a schematic diagram illustrating the catheter approached from the artery of the left arm, in which (A) illustrates a state of the catheter being inserted into an aortic arch from a left subclavian artery, and (B) illustrates a state of the catheter reaching a descending aorta.
[Fig. 9] Fig. 9 is a schematic diagram illustrating a state where the catheter approached from an artery of an arm is inserted into a celiac artery, in which (A) is a side view, and (B) is a cross-sectional view along an E-E line in (A).
[Fig. 10] Fig. 10 is a cross-sectional view illustrating another example of a state where the catheter approached from the artery of the arm is inserted into the celiac artery.
[Fig. 11] Fig. 11 is a schematic view illustrating a state where the catheter approached from the artery of the arm is engaged with the celiac artery.
[Fig. 12] Fig. 12 is a schematic view illustrating a state where the catheter approached from an artery of a right arm is inserted into an aorta.

### Description of Embodiments

Various embodiments according to the present invention will be described hereinafter with reference to the accompanying drawings. Dimensional ratios in the drawings are exaggerated for convenience of description and may differ from the actual ratios. Further, in the present description and the drawings, structural elements that have substantially the same function are denoted with the same reference numerals, and repeated explanation of these structural elements is omitted. In the present description, a side of a device to be inserted into a blood vessel is referred to as a "distal side", and a side of the device to be operated is referred to as a "proximal side".

### <First Embodiment>

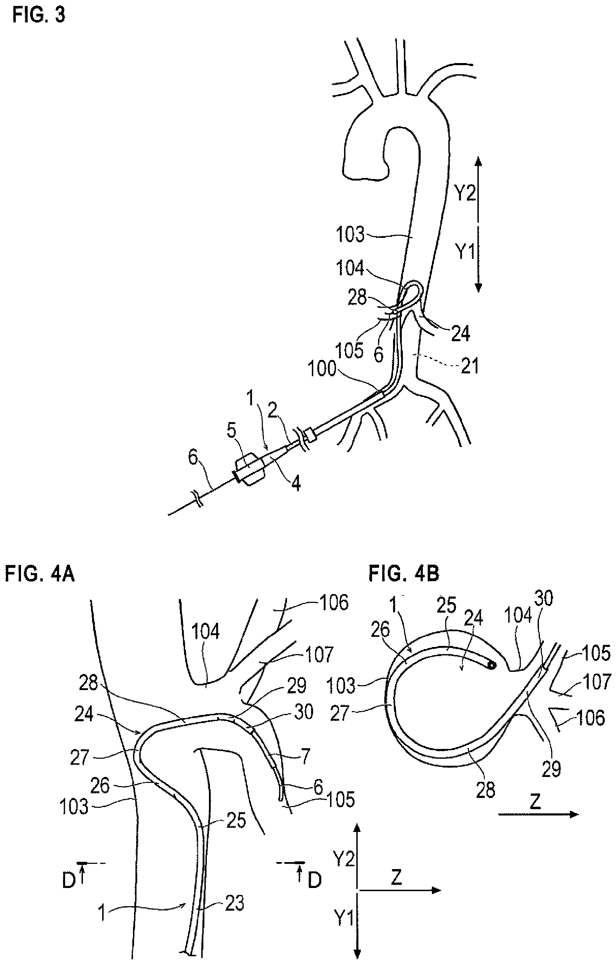

As illustrated in Fig. 3, a catheter 1 according to a first embodiment is a so-called guiding catheter for guiding a long device (for example, a microcatheter for treatment) to a common hepatic artery 105 which is a target site. The catheter 1 is introduced into a blood vessel from a leg artery 100 (for example, a femoral artery) of a patient, and is oriented to the vicinity of the common hepatic artery 105. The catheter 1 may be introduced into a blood vessel from an artery of an arm according to a shape of a blood vessel of the arm or an abdomen, and may be oriented to the vicinity of the common hepatic artery 105. The catheter 1 may be a so-called angiographic catheter, a guide wire support catheter, or a microcatheter.

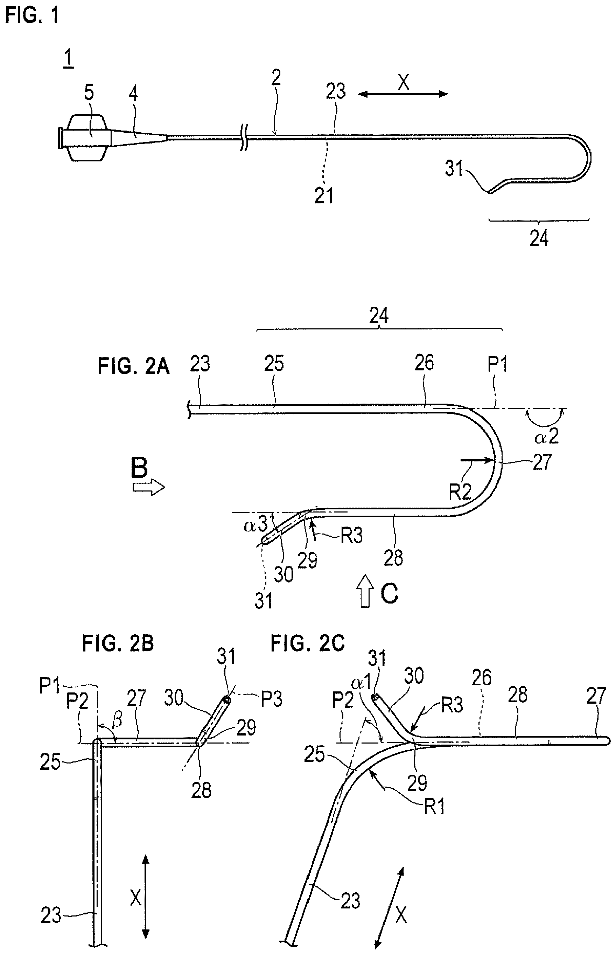

As illustrated in Figs. 1 and 2, the catheter 1 includes a tubular body 2, a hub 5 arranged on a proximal side of the tubular body 2, and an anti-kink protector 4.

The tubular body 2 is long and has flexibility. A lumen 21 extending from a proximal end to a distal end is formed in a substantially central portion of the tubular body 2.

A passage communicating with the lumen 21 is formed in the hub 5. The hub 5 is available to insert or remove, for example, a guide wire 6 or a therapeutic catheter 7 (see Fig. 3 and (A) in Fig. 4). In addition, the hub 5 is available to inject various liquids such as an X-ray contrast agent, a drug solution, an anticancer agent, and physiological saline, an embolic material such as embolic beads and an embolic coil, or a mixture thereof.

The anti-kink protector 4 is made of an elastic material. The anti-kink protector 4 covers a portion where the tubular body 2 and the hub 5 are connected to each other. Accordingly, the anti-kink protector 4 prevents the tubular body 2 from bending or kinking in the vicinity of this portion.

Next, the tubular body 2 will be described in detail. The tubular body 2 includes a substantially linear base portion 23, and a shaped portion 24 connected and shaped to a distal side of the base portion 23. The shaped portion 24 includes a first bending portion 25, a first intermediate portion 26, a second bending portion 27, a second intermediate portion 28, a third bending portion 29, and an extreme distal end portion 30.

The first bending portion 25 extends from a distal end of the base portion 23 to the distal side and is bent in a plane. The first intermediate portion 26 extends substantially linearly from a distal end of the first bending portion 25 to the distal side.

The second bending portion 27 extends from a distal end of the first intermediate portion 26 to the distal side and is bent in a plane. The second bending portion 27 is bent in a direction different from a direction in which the first bending portion 25 is bent. The second intermediate portion 28 extends substantially linearly from a distal end of the second bending portion 27 to the distal side. A direction in which the second bending portion 27 is bent is a clockwise direction from the proximal end to the distal end when the catheter 1 is viewed from the distal side in parallel to an extending direction X of the base portion 23. The direction in which the second bending portion 27 is bent may be a counterclockwise direction from the proximal end to the distal end when the catheter 1 is viewed from the distal side in parallel to the extending direction X.

The third bending portion 29 extends from a distal end of the second intermediate portion 28 to the distal side and is bent in a plane. The extreme distal end portion 30 extends substantially linearly from a distal end of the third bending portion 29 to the distal side. The lumen 21 is open at a distal end of the extreme distal end portion 30 to form a distal end opening 31. An axis of the extreme distal end portion 30 is not located on an extension line of an axis of the second intermediate portion 28.

An angle α1 formed by the first intermediate portion 26 with respect to an extension line extending from a portion of the base portion 23 on the distal side to a distal direction is more than 0° and less than 90°, preferably 40° to 85°, and more preferably 40° to 80°. When the angle α1 is less than 90°, it is possible to easily insert the catheter into a merging portion or a branching portion of a blood vessel, or a sharply bent blood vessel (hereinafter, these are referred to as bent portions), and it is easy to transmit a pushing force to the distal side.

An angle α2 formed by the second intermediate portion 28 with respect to an extension line extending from the first intermediate portion 26 to the distal direction is not particularly limited, and is substantially parallel to the extension line or more than 0° and less than 180°, preferably 100° to 175°, and more preferably 110° to 170°.

An angle α3 formed by the extreme distal end portion 30 with respect to an extension line extending from the second intermediate portion 28 to the distal direction is not particularly limited, and is more than 0° and less than 180°, preferably 5° to 80°, and more preferably 10° to 60°. The extreme distal end portion 30 is not positioned coaxially with the second intermediate portion 28.

The portion of the base portion 23 on the distal side, an axis of the first bending portion 25, and an axis of the first intermediate portion 26 are located on a first plane P1. An axis of the second bending portion 27 and an axis of the second intermediate portion 28 are located on a second plane P2. An axis of the third bending portion 29 and an axis of the extreme distal end portion 30 are located on a third plane P3. The axis of the first intermediate portion 26 located on the first plane P1 may also be located on the second plane P2. The axis of the second intermediate portion 28 located on the second plane P2 may also be located on the third plane P3.

The second plane P2 is a plane different from the first plane P1. An angle β at which the second plane P2 is inclined with respect to the first plane P1 on the distal side relative to the first bending portion 25 is more than 0° and less than 180°, preferably 30° to 150°, and more preferably 80° to 100°. Since the second plane P2 is different from the first plane P1, the shaped portion 24 including the first bending portion 25 and the second bending portion 27 can have a twisted shape so as to draw a three-dimensional spiral. Therefore, the shaped portion 24 of the catheter 1 easily follows an inner wall surface of the blood vessel, and a posture of the shaped portion 24 is easily stabilized in the blood vessel. In addition, when a proximal end portion of the catheter 1 is rotated in a direction in which the catheter 1 is wound toward the distal side, a rotational force is efficiently transmitted to the distal side, and thus the catheter 1 may be easily pushed in the distal direction.

The third plane P3 is a plane different from the first plane P1 and the second plane P2. The third plane P3 may coincide with the second plane P2, and preferably does not coincide with the second plane P2. Since the third plane P3 does not coincide with the second plane P2, the distal end opening 31 of the extreme distal end portion 30 can be oriented in a direction different from the bending of the second bending portion 27.

In the present embodiment, the third bending portion 29 is bent to a side away from the first plane P1 from the proximal side toward the distal side. Accordingly, the extreme distal end portion 30 extends from the proximal side toward the distal end opening 31 so as to be away from the first plane P1. The third bending portion 29 is bent to a side opposite to a side where the base portion 23 is located with respect to the second plane P2, from the proximal side toward the distal side. Therefore, the extreme distal end portion 30 extends to the side opposite to the side where the base portion 23 is located with respect to the second plane P2, from the proximal side toward the distal end opening 31. The third bending portion 29 may be bent to a side closer to the first plane P1 from the proximal side toward the distal side. In addition, the third bending portion 29 may be bent in a plane parallel to the first plane P1 from the proximal side toward the distal side. Further, the third bending portion 29 may be bent from the second plane P2 to the side opposite to the side where the base portion 23 is located, from the proximal side toward the distal side.

The first intermediate portion 26, the second intermediate portion 28, and the extreme distal end portion 30 may be substantially linear and may not be strictly linear.

When a minimum radius of curvature of the first bending portion 25 is R1, a minimum radius of curvature of the second bending portion 27 is R2, and a minimum radius of curvature of the third bending portion 29 is R3, R1 > R2 > R3 is satisfied. That is, R2 is smaller than R1, and R3 is smaller than R2. The minimum radius of curvature is a radius of curvature of a portion having the smallest radius of curvature in a bending portion.

In the tubular body 22, a reinforcing body may or may not be embedded in a range from the proximal end to a predetermined position in the distal direction. The reinforcing body is formed by braiding a plurality of strands into a tubular shape. The position of an extreme distal end of the reinforcing body is not particularly limited, and the reinforcing body may be arranged in the shaped portion 24 or the base portion 23.

An outer diameter of the tubular body 2 is preferably 1 mm (3 Fr) to 2.5 mm (6 Fr), and more preferably 1.3 mm (4 Fr) to 1.8 mm (5 Fr). An effective length of the tubular body 2 is preferably 800 mm to 1800 mm, more preferably 1000 mm to 1800 mm, and still more preferably 1000 mm to 1500 mm. It is preferable that the tubular body 2 can be appropriately selected depending on a patient, a blood vessel to which the catheter 1 is to be introduced, and the like. The effective length is a length of a portion that is insertable into a blood vessel, a sheath, or the like. In the present embodiment, the effective length is a length from an extreme distal end of the anti-kink protector 4 to an extreme distal end of the tubular body 2.

A distal tip that is more flexible than the proximal side may be arranged at a distal end portion of the tubular body 2. The distal tip is made of a material having high flexibility, such as rubber or elastomer. The number of layers and the constituent material of each layer constituting the tubular body 2, the presence or absence of the reinforcing body, and the like may be different along a longitudinal direction of the tubular body 2.

Alternatively, the tubular body 2 may include a flexible portion not provided with the reinforcing body on the distal side relative to a reinforcing portion where the reinforcing body is embedded. Since the catheter 1 includes the flexible portion, the catheter 1 is movable without damaging a blood vessel even in a bent, curved or branched blood vessel. A length of the flexible portion is preferably 500 mm or less, and more preferably 10 mm to 200 mm. As an example, in the case of the catheter 1 of 4 Fr, the length of the flexible portion is 150 mm. In the case of the catheter 1 of 5 Fr, the length of the flexible portion is 150 mm.

Examples of a constituent material of the tubular body 2 include various thermoplastic elastomers such as styrene-based, polyolefin-based, polyurethane-based, polyester-based, polyamide-based, polybutadiene-based, transpolyisoprene-based, fluororubber-based, and chlorinated polyethylene-based elastomers, polyetherketone, and polyimide-based ones, and a combination (polymer alloy, polymer blend, laminate, and the like) of one or two or more of above materials can be used. A low-friction material may be arranged on an inner peripheral surface of the tubular body 2 such that the guide wire 6 or another catheter may be easily inserted into the lumen 21. Examples of the low-friction material include fluorine-based resin materials such as polytetrafluoroethylene (PTFE). The tubular body 2 may contain an X-ray contrast material.

Next, a method of using the catheter 1 according to the present embodiment will be described by taking, as an example, a case where the catheter 1 is inserted into a blood vessel from the femoral artery 100, and is engaged with a celiac artery 104.

In a procedure, the operator inserts the guide wire 6 into the femoral artery 100, as illustrated in Fig. 3. Next, the operator pushes the catheter 1, in a state where the guide wire 6 is accommodated in the lumen 21, along the guide wire 6. Normally, a distal end of the guide wire 6 precedes the distal end of the catheter 1. Therefore, the shaped portion 24 of the catheter 1 is deformed into a shape close to a straight line by the guide wire 6 in the lumen 21. This causes the catheter 1 to move along a descending aorta 103 to an upper side Y2 (closer to a heart) and reach the vicinity of the celiac artery 104.

After the guide wire 6 and the catheter 1 reach the vicinity of an entrance of the celiac artery 104, the operator retracts the guide wire 6 to accommodate the guide wire 6 in the lumen 21 of the catheter 1. Accordingly, as illustrated in Fig. 4, the shaped portion 24 of the catheter 1 is restored to an original three-dimensionally bent shape. The operator can insert the distal end of the catheter 1 into the celiac artery 104 by utilizing the bent shape of the shaped portion 24. The celiac artery 104 generally extends from the descending aorta 103 to a front side Z (anterior). The common hepatic artery 105, a left gastric artery 106, and a splenic artery 107 are branched from the celiac artery 104. The common hepatic artery 105 generally extends from the celiac artery 104 to a lower side Y1 (a side close to a lower limb), and then extends obliquely upward to a liver. The left gastric artery 106 and the splenic artery 107 generally extend from the celiac artery 104 to the upper side Y2 (a side close to a head).

The first bending portion 25, the first intermediate portion 26, and the second bending portion 27 of the catheter 1 can be stably brought into contact with a blood vessel wall of the descending aorta 103 while drawing a three-dimensional spiral. At this time, the first bending portion 25 and/or the second bending portion 27 may be deformed such that the radius of curvature becomes larger or smaller. The first intermediate portion 26 may be bent. The second bending portion 27 of the catheter 1 is in contact with a blood vessel wall of the descending aorta 103 on a side opposite to the entrance of the celiac artery 104, and the second intermediate portion 28 and/or the third bending portion 29 are in contact with a blood vessel wall of the celiac artery 104. Accordingly, the second intermediate portion 28 and/or the third bending portion 29 of the catheter 1 are engaged with the celiac artery 104. In this state, the catheter 1 is provided with the third bending portion 29, so that the extreme distal end portion 30 can extend from the celiac artery 104 to the lower side Y1 and then extend to the common hepatic artery 105 extending obliquely upward to the liver. The third bending portion 29 and/or the extreme distal end portion 30 may or may not be in contact with the blood vessel wall of the common hepatic artery 105.

Thereafter, the therapeutic catheter 7 longer than the catheter 1 is inserted into the lumen 21 of the catheter 1 from the hub 5. The operator can easily insert the therapeutic catheter 7 into the common hepatic artery 105 by protruding from the distal end of the catheter 1. At this time, since the extreme distal end of the catheter 1 is oriented from the celiac artery 104 to the lower side Y1, it is possible to prevent the therapeutic catheter 7 from being erroneously inserted into the left gastric artery 106 or the splenic artery 107, which is simply oriented to the upper side Y2. Thereafter, the operator can insert the therapeutic catheter 7 into the common hepatic artery 105, and release an embolic agent or a drug solution through the therapeutic catheter 7. The catheter 1 alleviates the reaction caused by the insertion of the therapeutic catheter 7. Further, the catheter 1 is capable of applying a backup force to the therapeutic catheter 7 so as to hold the therapeutic catheter 7 at a desirable position. A medical device to be inserted into the catheter 1 may not be the therapeutic catheter 7.

### <Second Embodiment>

The catheter 1 according to a second embodiment is different from the catheter 1 according to the first embodiment in that the catheter 1 is introduced into a blood vessel from an artery of an arm of a patient, and is oriented to the vicinity of the common hepatic artery 105. The catheter 1 according to the second embodiment may be introduced into the blood vessel from the leg artery 100 of the patient, and may be oriented to the vicinity of the common hepatic artery 105.

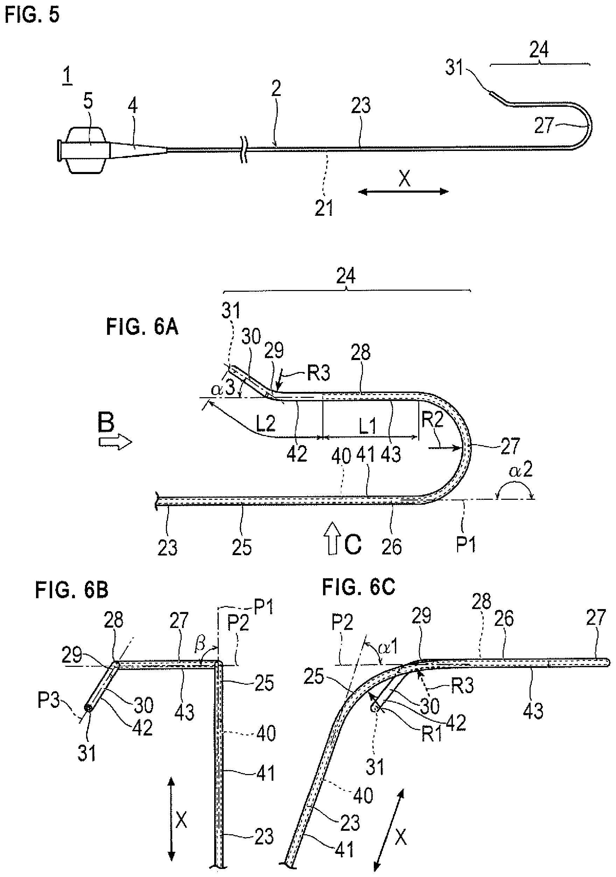

In the catheter 1 according to the second embodiment, as illustrated in Figs. 5 and 6, a direction in which the second bending portion 27 is bent is counterclockwise from the proximal end to the distal end when the catheter 1 is viewed in parallel to the extending direction X of the base portion 23 from the distal side. The third bending portion 29 is bent to the side where the base portion 23 is located with respect to the second plane P2, from the proximal side toward the distal side.

The outer diameter of the tubular body 2 is preferably 1 mm (3 Fr) to 2.5 mm (6 Fr), and more preferably 1.3 mm (4 Fr) to 1.8 mm (5 Fr). The effective length of the tubular body 2 is preferably 1000 mm to 1800 mm, more preferably 1100 mm to 1800 mm, and still more preferably 1000 mm to 1500 mm when the tubular body 2 is inserted from a radial artery of the arm. It is preferable that the tubular body 2 can be appropriately selected depending on a patient, a blood vessel to which the catheter 1 is to be introduced, and the like. The effective length is a length of a portion that is insertable into a blood vessel, a sheath, or the like. In the present embodiment, the effective length is a length from the extreme distal end of the anti-kink protector 4 to the extreme distal end of the tubular body 2. The effective length in the embodiment is 1200 mm or 1250 mm.

A distal tip that is more flexible than the proximal side may be arranged at a distal end portion of the tubular body 2. The distal tip is made of a material having high flexibility, such as rubber or elastomer. The number of layers and the constituent material of each layer constituting the tubular body 2, the presence or absence of a reinforcing body 40, and the like may be different along a longitudinal direction of the tubular body 2. The tubular body 2 may or may not include the reinforcing body 40.

Alternatively, the tubular body 2 may include a flexible portion 42 not provided with the reinforcing body 40 on the distal side of a reinforcing portion 41 where the reinforcing body 40 is embedded. The shaped portion 24 includes a distal side reinforcing portion 43 provided with the reinforcing body 40, and a flexible portion 42. The distal side reinforcing portion 43 is a portion of the reinforcing portion 41 on the distal side. Since the catheter 1 includes the flexible portion 42, the catheter 1 is movable without damaging a blood vessel even in a bent, curved or branched blood vessel. The length of the flexible portion 42 is preferably 500 mm or less, more preferably 10 mm to 200 mm, more preferably 10 mm to 50 mm, and still more preferably 11 mm to 47 mm. As an example, in the case of the catheter 1 of 4 Fr, the length of the flexible portion 42 is 23 mm. In the case of the catheter 1 of 5 Fr, the length of the flexible portion 42 is 23 mm.

Since other configurations are the same as those in the first embodiment, the description thereof will be omitted.

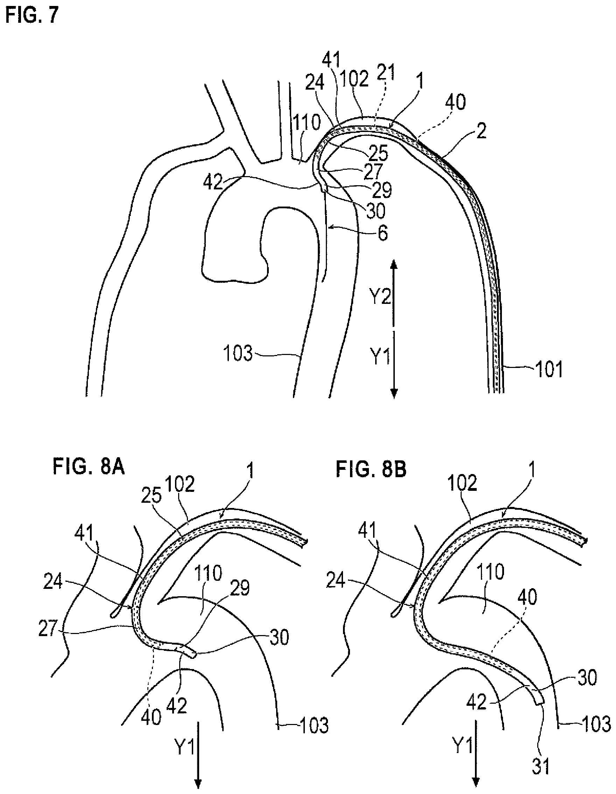

Next, a method of using the catheter 1 according to the second embodiment will be described by taking, as an example, a case where the catheter 1 is inserted into a blood vessel from a left arm artery 101, and is engaged with the celiac artery 104, as illustrated in Fig. 7. The artery of the arm to which the catheter 1 is approached is, for example, a distal radial artery, a conventional radial artery, an ulnar artery, a distal ulnar artery, a brachial artery, a snuff box radial artery, or the like.

In the procedure, the operator inserts the guide wire 6 into the left arm artery 101. Next, the operator pushes the catheter 1, in a state where the guide wire 6 is accommodated in the lumen 21, along the guide wire 6. Normally, the distal end of the guide wire 6 precedes the distal end of the catheter 1. Therefore, the shaped portion 24 of the catheter 1 is deformed into a shape close to a straight line by the guide wire 6 in the lumen 21.

As illustrated in Fig. 7 and (A) in Fig. 8, the guide wire 6 and the catheter 1 pass through the left subclavian artery 102 and proceed to the aortic arch 110. At a portion where the left subclavian artery 102 is connected to the aortic arch 110, the catheter 1 needs to bend greatly to move to the descending aorta 103. In order to cope with this, for example, the operator can temporarily retract the guide wire 6 and accommodate the guide wire 6 in the lumen 21 of the catheter 1. Accordingly, the shaped portion 24 of the catheter 1 is restored to the original three-dimensionally bent shape. The operator can proceed the distal end of the catheter 1 from the aortic arch 110 to the descending aorta 103 by utilizing the bent shape of the shaped portion 24. That is, the extreme distal end portion 30 of the catheter 1 can be oriented to the descending aorta 103 ahead of the aortic arch 110 at the aortic arch 110 that bends in a direction substantially opposite to a bending direction from the left subclavian artery 102 to the aortic arch 110. Subsequently, the operator can easily push the catheter 1 to proceed from the aortic arch 110 to the descending aorta 103, as illustrated in (B) in Fig. 8. Thereafter, the operator causes the guide wire 6 to protrude from the catheter 1. Subsequently, the operator further pushes the catheter 1 along the guide wire 6. The catheter 1 moves along the descending aorta 103 to the lower side Y1 (a side close to a lower limb) and reaches the vicinity of the celiac artery 104.

Since the catheter 1 includes the shaped portion 24 that is easily oriented from the left subclavian artery 102 to the descending aorta 103 through the aortic arch 110, the guide wire 6 may not be inserted. At this time, the operator can move the distal end portion of the catheter 1 to the descending aorta 103 only by the sensation of the hands without performing an X-ray fluoroscopy. Therefore, it is possible to reduce the exposure amount, which is one of disadvantages in a procedure to approach from an artery of a hand. Since it is possible to shorten the time of the procedure, the burden on the patient can be reduced, and the medical economy is improved.

In the case of approaching the catheter 1 from the left arm, it is usually performed with the left arm extended to the left with respect to the body. However, even in a state where the left arm is placed on an abdomen, the catheter 1 can move from the left subclavian artery 102 to the descending aorta 103 through the aortic arch 110. Therefore, the operator can perform the procedure on a right side of the body of the patient, and thus it is not necessary to change the layout of a catheter lab.

When the tubular body 2 includes the reinforcing portion 41 and the flexible portion 42, and the length of the flexible portion 42 is about 23 mm, a range in which the distal end portion of the catheter 1 is reinforced by the reinforcing body 40 is wide, so that the distal end of the catheter 1 is easily oriented to the descending aorta 103. Therefore, in the catheter 1 inserted from the arm artery 101 rather than the femoral artery 100, it is effective that the length of the flexible portion 42 is about 23 mm. In the case of the catheter 1 inserted from the femoral artery 100, it is not necessary to insert the catheter 1 into the descending aorta 103 from the subclavian artery 102. Therefore, when the length of the flexible portion 42 is, for example, about 150 mm, the distal end portion of the catheter 1 is flexible. In contrast, in the case of the catheter 1 inserted from the arm artery 101 rather than the femoral artery 100, the catheter 1 has a moderately hard structure including the flexible portion 42 having a length of about 23 mm in addition to the shaped portion 24, which is easily oriented from the left subclavian artery 102 to the descending aorta 103, and thus the catheter 1 is further easily moved to the descending aorta 103.

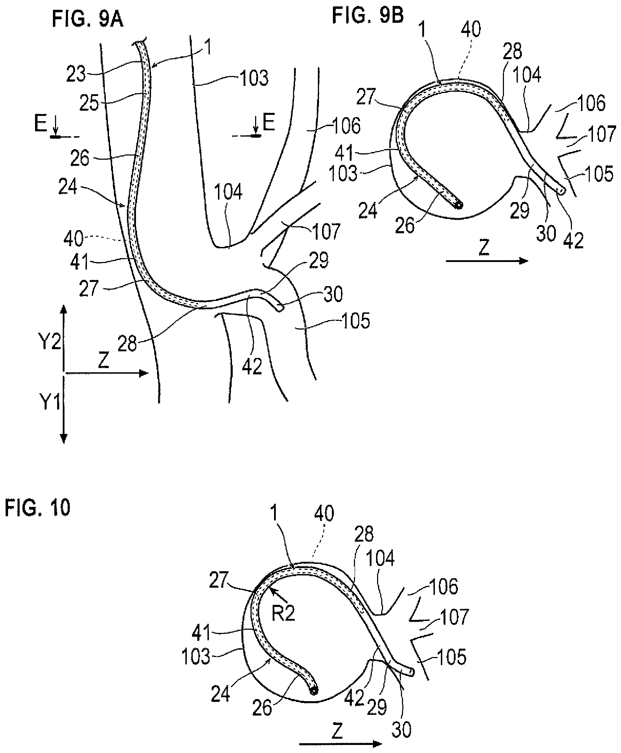

After the guide wire 6 and the catheter 1 reach the vicinity of the entrance of the celiac artery 104, the operator retracts the guide wire 6 to accommodate the guide wire 6 in the lumen 21 of the catheter 1. Accordingly, as illustrated in Fig. 9, the shaped portion 24 of the catheter 1 is restored to the original three-dimensionally bent shape. The operator can insert the distal end of the catheter 1 into the celiac artery 104 by utilizing the bent shape of the shaped portion 24.

The first bending portion 25, the first intermediate portion 26, and the second bending portion 27 of the catheter 1 can be stably brought into contact with a blood vessel wall of the descending aorta 103 while drawing a three-dimensional spiral. At this time, the first bending portion 25 and/or the second bending portion 27 may be deformed such that the radius of curvature becomes larger or smaller. The first intermediate portion 26 may be bent. For example, as illustrated in Fig. 10, even when the second bending portion 27 has a radius of curvature R2 of about 10 mm to 20 mm and is rapidly bent, it is possible to prevent kinking of the catheter 1 due to the presence of the reinforcing body 40. As illustrated in Figs. 9 and 10, the second bending portion 27 of the catheter 1 is in contact with the blood vessel wall on the side of the descending aorta 103 opposite to the entrance of the celiac artery 104, and the second intermediate portion 28 and/or the third bending portion 29 are in contact with the blood vessel wall of the celiac artery 104. The second bending portion 27 is in contact with a blood vessel wall of the celiac artery 104 on a rear side (opposite to the front side Z). Accordingly, the second intermediate portion 28 and/or the third bending portion 29 of the catheter 1 are engaged with the celiac artery 104. In this state, the catheter 1 is provided with the third bending portion 29, so that the extreme distal end portion 30 extends in a direction different from that of the second bending portion 27. Therefore, the extreme distal end of the catheter 1 can be oriented to the common hepatic artery 105, which extends from the celiac artery 104 to the lower side Y1. Alternatively, the extreme distal end of the catheter 1 can be easily proceeded to a proper hepatic artery (not shown) branching from the common hepatic artery 105. That is, the distal end opening 31 of the extreme distal end portion 30 of the catheter 1 can be oriented to the common hepatic artery 105 that is bent in a direction substantially opposite to a bending direction from the descending aorta 103 to the celiac artery 104. The third bending portion 29 and/or the extreme distal end portion 30 may or may not be in contact with the blood vessel wall of the common hepatic artery 105.

The flexible portion 42 is in contact with the blood vessel wall at the entrance of the celiac artery 104 on the opposite side of the descending aorta 103 with which the reinforcing portion 41 is in contact. That is, a portion where a distal end of the reinforcing portion 41 is located is floated in the descending aorta 103 without being into contact with the blood vessel wall. Since the reinforcing body 40 is not provided up to the third bending portion 29 and the extreme distal end portion 30, the distal end portion of the catheter 1 is flexible. Therefore, it is easy to control a direction of the extreme distal end portion 30 by changing a direction of the distal end portion of the catheter 1 along the guide wire 6 or by putting the guide wire 6 in and out of the catheter 1. A length along the axis of the shaped portion 24 (a length along the axis from the proximal end of the shaped portion 24 to the distal end opening 31) is the sum of a length L1 along the axis of the distal side reinforcing portion 43 and a length L2 along the axis of the flexible portion 42, and is preferably 30 mm to 50 mm. A ratio L2/(L1+L2) of the length L2 of the flexible portion 42 to the length (L1+L2) of the shaped portion 42 is preferably 0.36 to 0.93.

The catheter 1 may be coated with a lubricating coating in a proximal end direction along the axis from the distal end opening 31. The lubricating coating is, for example, formed in a range of 400 mm from the distal end opening 31 to the proximal end direction along the axis. Therefore, the operator can insert the catheter 1 deeply into the common hepatic artery 105. Examples of the constituent material of the lubricating coating include: a copolymer including an epoxy group-containing monomer such as glycidyl acrylate, glycidyl methacrylate, 3,4-epoxycyclohexylmethyl acrylate, 3,4-epoxycyclohexylmethyl methacrylate, β-methylglycidyl methacrylate, and allyl glycidyl ether, and a hydrophilic monomer such as N-methylacrylamide, N,N-dimethylacrylamide, and acrylamide; a (co)polymer including the hydrophilic monomer; a cellulose-based polymer material such as hydroxypropyl cellulose and carboxymethyl cellulose; a polysaccharide; polyvinyl alcohol; a methyl vinyl ether-maleic anhydride copolymer; a water-soluble polyamide; poly(2-hydroxyethyl (meth)acrylate); polyethylene glycol; polyacrylamide; and polyvinyl pyrrolidone.

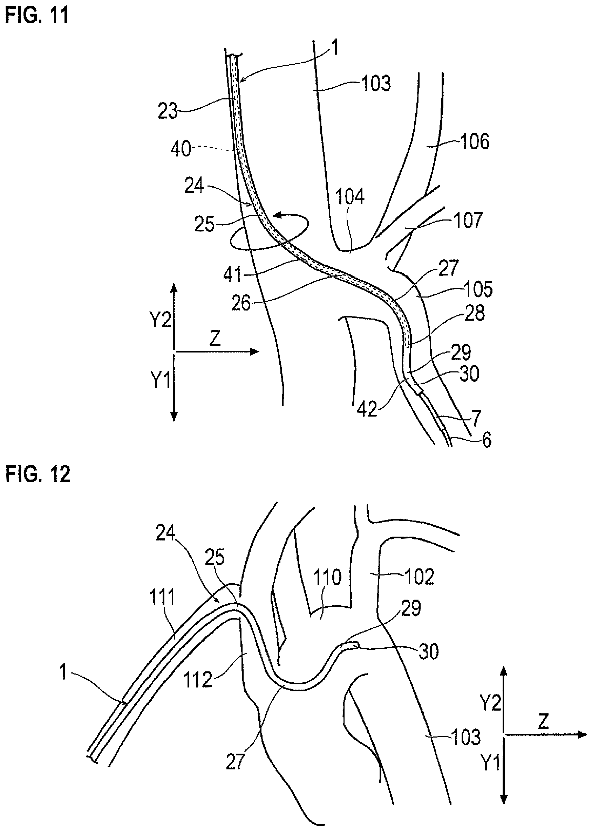

Thereafter, the operator can rotate the proximal end portion (for example, the hub 5) of the catheter 1 in a direction in which the shaped portion 24 is wound toward the distal side. Accordingly, the rotational force can be efficiently transmitted to the distal side along a spiral of the shaped portion 24, and as illustrated in Fig. 11, the extreme distal end portion 30 can be well pushed into the back of the common hepatic artery 105. Accordingly, the extreme distal end portion 30 can be arranged in the common hepatic artery 105 in a state where the shaped portion 24 of the catheter 1 is well engaged with a wall surface of the celiac artery 104. The distal end opening 31 opens from the common hepatic artery 105 toward the blood vessel ahead.

Thereafter, the therapeutic catheter 7 longer than the catheter 1 is inserted into the lumen 21 of the catheter 1 from the hub 5. The operator can easily insert the therapeutic catheter 7 into the common hepatic artery 105 by protruding from the distal end of the catheter 1. At this time, since the extreme distal end portion 30 of the catheter 1 is oriented to the lower side Y1 from the celiac artery 104 that extends to the lower side Y1 and then extends obliquely upward to the liver, it is possible to prevent the therapeutic catheter 7 from being erroneously inserted into the left gastric artery 106 or the splenic artery 107, which is simply oriented to the upper side Y2. Since the procedure thereafter is substantially the same as the case where the catheter 1 is approached from the femoral artery 100 described above, the description thereof will be omitted.

Next, a method of using the catheter 1 according to the second embodiment will be described by taking, as an example, a case where the catheter 1 is inserted into a blood vessel from a right arm artery 111, and is engaged with the celiac artery 104, as illustrated in Fig. 12.

In the procedure, the operator inserts the guide wire 6 into the right arm artery 111. Next, the operator pushes the catheter 1, in a state where the guide wire 6 is accommodated in the lumen 21, along the guide wire 6. Normally, the distal end of the guide wire 6 precedes the distal end of the catheter 1. Therefore, the shaped portion 24 of the catheter 1 is deformed into a shape close to a straight line by the guide wire 6 in the lumen 21.

The guide wire 6 and the catheter 1 pass through a brachiocephalic artery 112 and proceed to the aortic arch 110. At a portion where the brachiocephalic artery 112 is connected to the aortic arch 110, the catheter 1 needs to be bent greatly to move to the descending aorta 103. In order to cope with this, for example, the operator can temporarily retract the guide wire 6 and accommodate the guide wire 6 in the lumen 21 of the catheter 1. Accordingly, the shaped portion 24 of the catheter 1 is restored to the original three-dimensionally bent shape. The operator can proceed the distal end of the catheter 1 from the aortic arch 110 to the descending aorta 103 by utilizing the bent shape of the shaped portion 24. That is, the extreme distal end portion 30 of the catheter 1 can be oriented the descending aorta 103 ahead of the aortic arch 110 at the aortic arch 110 that bends in a direction substantially opposite to the bending direction from the brachiocephalic artery 112 to the aortic arch 110. Thereafter, the operator causes the guide wire 6 to protrude from the catheter 1. Subsequently, the operator further pushes the catheter 1 along the guide wire 6. Accordingly, the catheter 1 can be easily proceeded to the descending aorta 103 from the aortic arch 110. The catheter 1 moves along the descending aorta 103 to the lower side Y1 (a side close to a lower limb) and reaches the vicinity of the celiac artery 104. Since the engagement of the catheter 1 with the celiac artery 104 is almost the same as the case where the catheter 1 is approached from the left arm artery 101, the description thereof will be omitted. In the catheter 1, the catheter 1 can be proceeded quite easily with respect to a Type I in which a difference in height between the brachiocephalic artery and an aortic root is equal to or less than a diameter of a common carotid artery or a Type II in which the difference in height is more than an equal multiple and equal to or less than three times. Further, even in the case of a type III case in which the catheter 1 is arranged on the lower side Y1 from the common carotid artery-aortic root, the catheter 1 can be proceeded from the brachiocephalic artery 112 to the descending aorta 103 through the aortic arch 110.

In general, in the case of a Type III blood vessel, a method of firstly inserting a pigtail-type guiding catheter, inserting the guide wire 6 into a descending aorta, and replacing the guiding catheter with a cobra-type guiding catheter that is engageable with a celiac artery along the guide wire 6 is performed. In this case, since it is necessary to replace the guiding catheter, there is a problem that the time for the procedure is long and the number of catheters used increases. In contrast, in the first and second embodiments, effects that the time for the procedure can be shortened and the number of guiding catheters used can be reduced to reduce the cost are achieved.

As described above, the catheter 1 according to the first and second embodiments is the catheter 1 including the tubular body 2 that communicates from the proximal end to the distal end, in which the tubular body 2 includes the substantially linear base portion 23 and the shaped portion 24 connected to the distal side of the base portion 23, and the shaped portion 24 includes the first bending portion 25 that is bent at the distal side relative to the base portion 23, the first intermediate portion 26 that is located on the distal side relative to the first bending portion 25, the second bending portion 27 that is bent at the distal side relative to the first intermediate portion 26, the second intermediate portion 28 that is located on the distal side relative to the second bending portion 27, the third bending portion 29 that is bent at the distal side relative to the second intermediate portion 28, and the extreme distal end portion 30 that is located on the distal side relative to the third bending portion 29 and has the distal end opening 31 formed therein, the first bending portion 25 and the first intermediate portion 26 are located on the first plane P1, the second bending portion 27 and the second intermediate portion 28 are located on the second plane P2 different from the first plane P1, the angle α1 formed by the first intermediate portion 26 with respect to the extension line extending from the portion of the base portion 23 on the distal side to the distal direction is more than 0° and less than 90°, and the axis of the extreme distal end portion 30 is separated from the extension line of the axis of the second intermediate portion 28.

In the catheter 1 configured as described above, since the angle α1 formed by the first intermediate portion 26 with respect to the extension line extending from the portion of the base portion 23 on the distal side to the distal direction is more than 0° and less than 90°, it is easy to transmit the pushing force to the distal side. In addition, since the axis of the extreme distal end portion 30 is away from the extension line of the axis of the second intermediate portion 28, the extreme distal end portion 30 can be oriented in a desirable direction in a blood vessel ahead of a bent portion of the blood vessel. Accordingly, the catheter 1 can be easily proceeded to the blood vessel ahead of the bent portion of the blood vessel. In addition, the catheter 1 can effectively assist other devices in proceeding into the blood vessel ahead of the bent portion of the blood vessel.

The third bending portion 29 may be bent to the side away from the first plane P1 from the proximal side toward the distal side. Accordingly, the catheter 1 can be oriented in a desirable direction with respect to the blood vessel oriented in a predetermined direction in the blood vessel ahead of the bent portion of the blood vessel. Since the third bending portion 29 is bent in a direction away from the first plane P1, the catheter 1 is likely to transmit the pushing force from the proximal side to the distal end of the catheter 1, as compared with a case where the catheter 1 is bent in a direction closer to the first plane P1. When the third bending portion 29 is bent in the direction away from the first plane P1, the third bending portion 29 is likely to have a structure of being bent to a side substantially opposite to the second bending portion 27. Therefore, when the second bending portion 27 passes the bent portion of the blood vessel, the extreme distal end portion 30 can be oriented to a side substantially opposite to a bending direction of the bent portion of the blood vessel. Therefore, for example, when the blood vessel ahead of the bent portion of the blood vessel is bent in a direction different from the bending direction of the bent portion, the extreme distal end portion 30 can be oriented in an appropriate direction with respect to the blood vessel ahead of the bent portion. For example, when the catheter 1 is approached from the left arm artery 101, the catheter 1 can pass a bent portion between the left subclavian artery 102 and the aortic arch 110, and can be oriented to the descending aorta 103 that extends downward while bending in a substantially opposite direction ahead of the bent portion. For example, the catheter 1 can pass a bent portion between the descending aorta 103 and a side branch (for example, the celiac artery 104) and can easily reach a blood vessel (for example, the common hepatic artery 105) bent in a substantially opposite direction ahead of the bent portion. For example, a direction in which the blood vessel ahead of the bent portion of the blood vessel is bent with respect to the bending direction of the bent portion is not particularly limited. Therefore, even when the blood vessel ahead of the bent portion of the blood vessel is bent in substantially the same direction as the bending direction of the bent portion, the catheter 1 can be oriented in a desirable direction in the blood vessel ahead of the bent portion.

The third bending portion 29 may be bent to the side where the base portion 23 is located with respect to the second plane P2, from the proximal side toward the distal side. Accordingly, when the first bending portion 25, the first intermediate portion 26, and the second bending portion 27 are deformed so as to draw a three-dimensional spiral in the blood vessel, the extreme distal end portion 30 oriented by the third bending portion 29 can be oriented in a desirable direction with respect to the blood vessel oriented in a predetermined direction.

The third bending portion 29 may be bent to a side opposite to the side where the base portion 23 is located with respect to the second plane P2, from the proximal side toward the distal side. Accordingly, when the first bending portion 25, the first intermediate portion 26, and the second bending portion 27 are deformed so as to draw the three-dimensional spiral in the blood vessel, the extreme distal end portion 30 oriented by the third bending portion 29 may be oriented in a desirable direction with respect to the blood vessel oriented in a predetermined direction.

A direction in which the second bending portion 27 is bent is the counterclockwise direction from the proximal end to the distal end when the catheter 1 is viewed from the distal side in parallel to the extending direction X of the base portion 23. Accordingly, the catheter 1 can pass the bent portion of the blood vessel by utilizing counterclockwise bending of the second bending portion 27. Further, the operator can effectively transmit a rotational force to the shaped portion 24 of the catheter 1 by applying the rotational force to the proximal end portion of the catheter 1 in a direction (a counterclockwise direction) in which the second bending portion 27 is bent to the distal side. When the three-dimensional shaped portion 24 of the catheter 1 rotates, the catheter 1 can easily pass the bent portion of the blood vessel.

A direction in which the second bending portion 27 is bent is a clockwise direction from the proximal end to the distal end when the catheter 1 is viewed from the distal side in parallel to the extending direction X of the base portion 23. Accordingly, the catheter 1 may pass the bent portion of the blood vessel by utilizing the counterclockwise curve of the second bending portion 27. Further, the operator can effectively transmit the rotational force to the shaped portion 24 of the catheter 1 by applying the rotational force to the proximal end portion of the catheter 1 in a direction (a clockwise direction) in which the second bending portion 27 is bent to the distal side. When the three-dimensional shaped portion 24 of the catheter 1 rotates, the catheter 1 may easily pass the bent portion of the blood vessel.

The minimum radius of curvature R2 of the second bending portion 27 is smaller than the minimum radius of curvature R1 of the first bending portion 25, and the minimum radius of curvature R3 of the third bending portion 29 is smaller than the minimum radius of curvature R2 of the second bending portion 27. Therefore, when the catheter 1 is proceeded from the left subclavian artery 102 to the descending aorta 103, the first bending portion is in contact with an upper side of the left subclavian artery 102, and the second bending portion 27 is in contact with a blood vessel wall of the aortic arch 110 on an ascending aorta side and a chest side. Since the entire shaped portion 24 is in a stable state and the third bending portion 29 is oriented to a dorsal side of the descending aorta 103 in a state of being floated in the aorta, the catheter 1 can be easily proceeded to the descending aorta 103 without the guide wire 6.

The present invention is not limited to the embodiments described above, and various modifications can be made by those skilled in the art within a scope of the technical idea of the present invention. For example, the catheter 1 may have a different size depending on an inner diameter of a blood vessel. Alternatively, the flexible portion 42 may be one in which the hardness of a resin is increased stepwise from the distal end to the proximal end, one in which the thickness or pitch of a reinforcing body is changed, or one in which both are combined.

The blood vessel with which the catheter 1 is to be engaged is not limited to the celiac artery 104. For example, the blood vessel with which the catheter 1 is to be engaged may be a superior mesenteric artery, an inferior mesenteric artery, a renal artery, a lumbar artery, a splenic artery, a left gastric artery, a testis artery, a common iliac artery, an internal iliac artery, a prostate artery, a uterine artery, an external iliac artery, a coronary artery, or the like. The catheter 1 may be used to guide a device to be inserted into a carotid artery. The catheter 1 may well pass through a blood vessel that is largely bent between a right subclavian artery and a right common carotid artery.

The catheter 1 may be an outer guiding catheter into which an inner catheter is to be inserted. The inner catheter has a linear shape. Alternatively, the catheter 1 may be an inner catheter or a dilator that is to be inserted into the outer guiding catheter. The inner catheter may include a flexible distal end portion and a rigid base portion.

The application is based on Japanese Patent Application No. 2019-179471, filed on September 30, 2019, the disclosure of which is referenced and incorporated as a whole.

### Reference Signs List

- 1: catheter
- 2: tubular body
- 21: lumen
- 23: base portion
- 24: shaped portion
- 25: first bending portion
- 26: first intermediate portion
- 27: second bending portion
- 28: second intermediate portion
- 29: third bending portion
- 30: extreme distal end portion
- 31: distal end opening
- 100: leg artery
- 101: left arm artery
- 102: left subclavian artery
- 103: descending aorta
- 104: celiac artery
- 105: common hepatic artery
- 110: aortic arch
- 111: right arm artery
- P1: first plane
- P2: second plane
- P3: third plane

## Claims

1. A catheter comprising:
a tubular body that communicates from a proximal end to a distal end, wherein
the tubular body includes a substantially linear base portion and a shaped portion connected to a distal side of the base portion,
the shaped portion includes
a first bending portion that is bent at a distal side relative to the base portion,
a first intermediate portion that is located on a distal side relative to the first bending portion,
a second bending portion that is bent at a distal side relative to the first intermediate portion,
a second intermediate portion that is located on a distal side relative to the second bending portion,
a third bending portion that is bent at a distal side relative to the second intermediate portion, and
an extreme distal end portion that is located on a distal side relative to the third bending portion and has a distal end opening formed therein,
the first bending portion and the first intermediate portion are located on a first plane,
the second bending portion and the second intermediate portion are located on a second plane different from the first plane,
an angle formed by the first intermediate portion with respect to an extension line extending from a portion of the base portion on the distal side to a distal direction is more than 0° and less than 90°, and
an axis of the extreme distal end portion is separated from an extension line of an axis of the second intermediate portion.

2. The catheter according to claim 1, wherein
the third bending portion is bent to a side away from the first plane from a proximal side toward the distal side.

3. The catheter according to claim 1 or 2, wherein
the third bending portion is bent to a side where the base portion is located with respect to the second plane, from the proximal side toward the distal side.

4. The catheter according to claim 1 or 2, wherein
the third bending portion is bent to a side opposite to a side where the base portion is located with respect to the second plane, from the proximal side toward the distal side.

5. The catheter according to any one of claims 1 to 4, wherein
a direction in which the second bending portion is bent is a counterclockwise direction from the proximal end to the distal end when the catheter is viewed from the distal side in parallel to an extending direction of the base portion.

6. The catheter according to any one of claims 1 to 4, wherein
a direction in which the second bending portion is bent is a clockwise direction from the proximal end to the distal end when the catheter is viewed from the distal side in parallel to an extending direction of the base portion.

7. The catheter according to any one of claims 1 to 6, wherein
a minimum radius of curvature of the second bending portion is smaller than a minimum radius of curvature of the first bending portion, and a minimum radius of curvature of the third bending portion is smaller than the minimum radius of curvature of the second bending portion.
